# EUROPEAN PATENT APPLICATION

(11) **EP 0 972 508 A1**
(43) Date of publication of application: **19.01.2000**
(21) Application number: 98945608.2
(22) Date of filing: 05.10.1998
(51) Int. Cl.: A61K 7/00, A61K 7/48, A61K 35/78

(54) **EXTRACELLULAR MATRIX PRODUCTION PROMOTER**

(30) Priority: 07.10.1997 JP 29032897; 07.10.1997 JP 29032997
(71) Applicant: SHISEIDO COMPANY LIMITED, Chuo-ku, Tokyo 104-8010 (JP)
(72) Inventor: NAKAYAMA, Yasukazu Shiseido Research Center, Yokohama-shi, Kanagawa 223-8553 (JP); AKUTSU, Nobuko Shiseido Research Center, Yokohama-shi, Kanagawa 236-8643 (JP); NISHIYAMA, Toshio Shiseido Research Center, Yokohama-shi, Kanagawa 236-8643 (JP); HORII, Izumi Shiseido Research Center, Yokohama-shi, Kanagawa 236-8643 (JP)
(74) Representative: Henkel, Feiler, Hänzel
(86) International application number: JP9804482
(87) International publication number: WO9917715

(57) **Abstract**

An agent which promotes the production of extracellular matrix; more particularly, acidic mucopolysaccharides such as hyaluronic acid and fibrous proteins such as collagen, and thus exhibits the effects of activating the extracellular matrix, normalizing skin tissue, and efficiently preventing skin aging by reinforcing the binding between the epidermis and the dermis. This agent contains as active ingredients the extracts of plants belonging to the genus *Bupleurum* such as *B. falcatum Linne* (Mishima Saiko), or saikosaponin and saikogenin contained in these extracts. Use of this agent as a skin preparation for external use, etc. makes it possible to promote the production of the above acidic mucosaccharides, fibrous proteins, etc. in fibroblasts, etc.

## Description

### Technical Field

The present invention relates to an agent such as a skin treatment preparation for external use (hereinafter referred to as a skin preparation for external use). More particularly, the present invention relates to an agent such as a skin preparation for external use that promotes production of extracellular matrix that constitutes the dermis and comprises acidic mucosaccharides such as hyaluronic acid and fibrous proteins such as collagen.

### Background Art

The dermis is connective tissue that exists under the epidermis. The density of cells in the dermis is lower than that in the epidermis, and the dermis is rich in extracellular space which is filled with extracellular matrix; i.e., giant molecules forming a network structure.

The extracellular matrix is produced from sites such as fibroblasts in the dermis and comprises acidic mucopolysaccharides such as hyaluronic acid and dermatan sulfate, and fibrous proteins such as collagen and elastin.

The extracellular matrix directly relates to elasticity, tension, moistness, and metabolism of the skin. Therefore, when the production of the extracellular matrix from sites such as fibroblasts decreases, elasticity and moistness of the skin decrease to thereby easily cause wrinkles, fine wrinkles, and rough skin, all of which are factors of skin aging.

Presently, efforts are being carried out to discover means for maintaining tension and moistness of the skin and providing young-looking skin by promoting the production of the extracellular matrix.

As described hereinabove, an object of the present invention is to solve the above problem by providing an agent such as a skin preparation for external use that can prevent skin aging phenomena represented by wrinkles, fine wrinkles, as well as rough skin, without involving any problems of safety in use, by finding a substance for promoting production of the extracellular matrix and making use of the substance as an active ingredient of the agent.

### Disclosure of the Invention

The present Inventors have conducted earnest studies to attain the object, and have found that an extract of plants belonging to the genus *Bupleurum* such as *B. falcatum Linne* or a substance contained the extract such as saikosaponin acts on epidermal cells and fibroblasts in the skin to thereby promote production of extracellular matrix, activate the extracellular matrix, and normalize skin tissue. The present invention has been accomplished based on this finding.

Accordingly, the present invention provides an extracellular matrix production promoting agent comprising an extract of plants belonging to the genus *Bupleurum* as an active ingredient (hereinafter may be referred to as "the agent according to the present invention"); *inter alia*, the agent comprising an extract of *B. falcatum Linne* (Mishima Saiko) as a plant belonging to the genu*s Bupleurum.*

### Brief Description of Drawings

Fig. 1 is a graph showing test results of hyaluronic acid production in human epidermal cells.
Fig. 2 is a graph showing test results of hyaluronic acid production in fibroblasts originating from human skin.
Figs. 3A through 3C are photographs showing the morphology of organisms generated in a test of VII-type collagen production in human epidermal cells.
Figs. 4A through 4C are photographs showing the morphology of organisms generated in a test of VII-type collagen production in fibroblasts originating from human skin.

### Best Mode for Carrying out the Invention

Modes for carrying out the present invention will next be described.

The agent according to the present invention may comprise an extract of plants belonging to the genus *Bupleurum* as an active ingredient.

No particular limitation is imposed on the plants belonging to the genus *Bupleurum* for providing an extract that may be employed as an active ingredient of the agent according to the present invention, so long as they contain saikosaponin or saikogenin.

Examples of the plants belonging to the genus *Bupleurum* that serve as a source of the extract include *Bupleurum falcatum Linne* (the name listed in *Japanese Pharmacopoeia*, 13th revision), also-called Mishima Saiko, *Bupleurum chinese DC*., also-called Manshu Mishima Saiko, *Bupleurum scorzonerifolium Willd*., also-called Okumishima Saiko, *Bupleurum marginatum Wall. ex DC*., *Bupleurum Komarovianum Lincz., Bupleurum longiradiatum Turcz., Bupleurum sibiricum Vest, Bupleurum longicaule Wall. ex DC., Bupleurum tenue Buch.-Ham ex D. Don, Bupleurum aureum Fisch.,* so-called *Sen*you Saiko, and *Bupleurum multinerve DC.*

In the above plants, no particular limitation is imposed on the portions to be used for providing the extract, so long as they contain saikosaponin or saikogenin. For example, the above-described *B. falcatum Linne* (hereinafter referred to as "Mishima Saiko") is known to contain saikosaponin and saikogenin in the root portion, and in this case the root portion is preferably selected.

Although natural plants can be used as the plants for providing the extract, there may also be used artificially cultured plants, such as callus, which is derived by culturing a specific portion of the above-described plants so as to enhance the efficiency in producing saikosaponin and/or saikogenin, or a plant obtained by organ-culturing a specific portion of the cell-differentiated callus.

In addition, there may also be used gene-transferred strains and cell-fused strains which are produced through a gene engineering method in order to produce saikosaponin or saikogenin in large amounts.

When Mishima Saiko is preferably selected as a plant which produces saikosaponin and/or saikogenin, protoplasts thereof are preferably cultured in a culture medium containing at least one vitamin or organic acid selected from among nicotic acid, pyridoxine hydrochloride, calcium d-pantothenate, p-aminobenzoic acid, choline chloride, ascorbic acid, vitamin A, vitamin D₃, vitamin B₁₂, sodium pyruvate, citric acid, sodium malate, fumaric acid, and Casamino acid (See Japanese Patent Application Laid-Open (*kokai*) No. 2-245180).

When the root of Mishima Saiko is organ-cultured, the root is preferably cultured in a culture medium, the amount of saccharide ingredients (sucrose, glucose, fructose, etc.) in the medium being limited to less than 2.0 wt.% based on the total amount of the medium, until the number of the lateral roots does not substantially increase during continuation of organ-culturing, in view of enhancement in production efficiency of saikosaponin during organ-culturing (See Japanese Patent Application Laid-Open (*kokai*) No. 5-23069). Moreover, there is preferably added to the culture medium phenolic derivatives such as coumarin, m-hydroxybenzoic acid, o-hydroxyphenylacetic acid, p-hydroxyphenylacetic acid, o-coumarinic acid, chlorogenic acid, phloroglucinol, and umbelliferone, in that the derivatives enhance the production efficiency of saikosaponin during organ-culturing (See Japanese Patent Application Laid-Open (*kokai*) No. 5-184379).

When a gene which can enhance production of saikosaponin, such as a saikosaponin-synthesizing gene, is transferred to a plant belonging to the genus *Bupleurum* such as Mishima Saiko, DNA containing the corresponding genetic code and protoplasts of the transfer target plant belonging to the genus *Bupleurum* are preferably placed together with application of electric pulse or addition of a cell-fusing agent, in that a target gene can be effectively transferred (See Japanese Patent Application Laid-Open (*kokai*) No. 6-90771).

An extract of the plant belonging to the genus *Bupleurum* can be obtained through a generally known method for obtaining such an extract; e.g., solvent extraction. In the present description, the term "extract" refers to extract originating from crushed plants as well as extract originating from a medium obtained through tissue-culturing, such as organ-culturing.

Examples of the solvents for extraction include water such as purified water; primary lower alcohols such as methanol and ethanol; primary higher alcohols such as oleyl alcohol, stearyl alcohol, and octyldodecanol; polyols such as ethylene glycol, propylene glycol, glycerin, and 1,3-butylene glycol; ketones such as acetone; esters such as ethyl acetate; and hydrocarbon solvents such as hexane, chloroform, and benzene. The solvents may be used singly or in combination of two or more species. Of these, water, ethanol, glycerin, 1,3-butylene glycol, and mixtures of two or more species are appropriate in that they have wide applicability to organisms.

Thus, an extract of a plant belonging to the genus *Bupleurum* (hereinafter may be referred to simply as "extract") can be obtained.

No particular limitation is imposed on the amount of the extract incorporated into the agent according to the present invention, and the amount may be dependent on the specific extraction material and the form of the agent, and, in addition, a component of extracellular matrix whose production is particularly to be promoted.

For example, when the extract of the root of Mishima Saiko is used as an active ingredient of the agent according to the present invention, the amount of the extract is appropriately selected from a preferable range as wide as 0.00001-10.0 wt.% as reduced to the solid content, based on the total amount of the agent.

Furthermore, saikosaponin and/or saikogen known to be contained in the above-described extract may be incorporated as active ingredients into the agent according to the present invention.

Saikosaponin obtained by drying the root of Mishima Saiko is known as a predominant pharmacological component of "Saiko," which is an important source of traditional Chinese remedies and has been used from ancient times as an important component of antipyretic, detoxicative, analgesic, tonic, and anti-inflammatory drugs.

The saikosaponin is an oleanan-type triterpenoid saponin, and 13 species thereof, including a, c, d, b1, and b2, have been isolated and structurally determined.

These saikosaponin species isolated through a generally known method may be incorporated into the agent according to the present invention.

Moreover, saikosaponin which is obtained through a method for large-scale production may also be incorporated into the agent according to the present invention (the amount of saikosaponin in the natural root of Mishima Saiko is only about 0.5-1.5 wt.%.).

For example, target saikosaponin species can be obtained in large quantities by causing an adsorbent to adsorb the above extract containing saikosaponin and treating the adsorbed adsorbent in a variety of manners.

More specifically, the method comprises the steps of bringing the above extract into contact with an adsorbent to thereby cause the adsorbent to adsorb saikosaponin, and removing from the adsorbent the components contained in the extract other than saikosaponin (adsorption steps); and eluting saikosaponin adsorbed by the adsorbent to thereby obtain purified natural saikosaponin (saikosaponin existing in a natural product, represented by one having a double bond exclusively at the 13 position: saikosaponin a, saikosaponin c, and saikosaponin d).

Alternatively, after completion of the adsorption of the above extract by the adsorbent, the adsorbed saikosaponin may be brought into contact with Fe(III) ions (e.g., a solution of Fe(III) ions containing an Fe(III) salt such as ferric chloride (FeCl₃) may be employed), to thereby obtain saikosaponin having a dienic structure (e.g., saikosaponin having double bonds at the 13 and 14a positions, such as saikosaponin b1, saikosaponin h, or saikosaponin b2).

Saikosaponin having a dienic structure obtained by converting natural-type saikosaponin a corresponds to saikosaponin b1. Similarly, conversion of saikosaponin c and saikosaponin d yields saikosaponin h and saikosaponin b2, respectively.

Examples of the adsorbents which are used in the above steps include active carbon, styrene-divinylbenzene synthetic adsorbents, and acrylic synthetic adsorbents. Other examples include synthetic adsorbents formed by combining silica gel with hydrohpobic group, polyamide gel, and modified dextran gel.

A target saikosaponin can be obtained by eluting the adsorbed saikosaponin adsorbed by the adsorbent with an appropriate eluent and, if necessary, purifying through a method such as high performance liquid chromatography.

The saikosaponin species other than those whose preparation methods are described above, such as saikosaponin e, saikosaponin f, and saikosaponin g can be obtained by generally known methods.

Into the agent according to the present invention, there may be incorporated any of the above saikosaponin a, saikosaponin b1, saikosaponin b2, saikosaponin c, saikosaponin d, saikosaponin e, saikosaponin f, saikosaponin g, and saikosaponin h.

Of these, saikosaponin a, saikosaponin b1, saikosaponin b2, saikosaponin c, and saikosaponin d are preferred as an active ingredient of the agent according to the present invention, in view of remarkably strong production promoting effect on hyaluronic acid.

No particular limitation is imposed on the amount of the above saikosaponin incorporated into the agent according to the present invention, and the amount may be dependent on the selected saikosaponin species and the form of the agent, and, in addition, the component of extracellular matrix whose production is particularly to be promoted. The amount is appropriately selected from a preferable range as wide as 0.000001-10.0 wt.% based on the total amount of the agent.

Into the agent according to the present invention, there may be also incorporated saikogenin, which is a genin moiety of the above-described saikosaponin.

Saikogenin is a genin moiety of the above-described saikosaponin; i.e., a non-saccharide moiety of saikosaponin.

Specifically, into the agent according to the present invention, there may be incorporated any of saikogenins such as saikogenin A, which is a genin moiety of saikosaponin b1; saikogenin C, which is a genin moiety of saikosaponin h; saikogenin D, which is a genin moiety of saikosaponin b2; saikogenin E, which is a genin moiety of saikosaponin c; saikogenin F, which is a genin moiety of saikosaponin a; and saikogenin G, which is a genin moiety of saikosaponin d.

These saikogenin species can be obtained by hydrolyzing the corresponding saikosaponin species.

Specifically, the above saikogenin species can be obtained through desaccharide reaction under an acidic condition, such as presence of hydrochloric acid or sulfuric acid.

No particular limitation is imposed on the amount of the saikogenin incorporated into the agent according to the present invention, and the amount may be dependent on the selected saikogenin species and the form of the agent, and, in addition, the component of extracellular matrix whose production is particularly to be promoted. The amount is appropriately selected from a preferable range as wide as 0.000001-10.0 wt.% based on the total amount of the agent.

The thus-produced active ingredients; i.e., an extract of plants belonging to the genu*s Bupleurum*, saikosaponin, and saikogenin, are incorporated into the agent singly or in combination of two or more species, to thereby provide the agent of the present invention. The agent can promote production of extracellular matrix, and, on the basis of the promotion thereof, normalize the skin tissue to thereby exhibit excellent prevention effect on skin aging; e.g., prevention of wrinkles and fine wrinkles or enriching moistness of the skin.

As described above, the extracellular matrix whose production is promoted by the agent according to the present invention is predominantly produced from fibroblasts. Specifically, the matrix comprises acidic mucopolysaccharides such as hyaluronic acid and dermatan sulfate, and fibrous proteins such as collagen, elastin, fibronectin, and laminin.

Acidic mucopolysaccharide retains a large amount of water in the molecule to exist as a gel, and water in the gel plays a role in moving nutrition, metabolites, hormones, etc. from blood vessels to cells in tissue. Therefore, if the production of acidic mucopolysaccharides is promoted in extracellular matrix, not only the moistness of the skin increases, but also the metabolism of the skin is promoted, to thereby prevent skin aging.

Fibrous protein plays a role in maintaining the shape of tissues and imparting elasticity to the skin. Therefore, if the production of fibrous proteins is promoted in extracellular matrix, the skin becomes elastic to prevent wrinkles and fine wrinkles.

The agent according to the present invention enables promotion of the production of both components of the extracellular matrix.

In this sense, the agent according to the present invention serves as "an acidic mucopolysaccharide production promoting agent" and as "a fibrous protein production promoting agent."

Particularly, the agent according to the present invention remarkably promotes the production of hyaluronic acid among the acidic mucopolysaccharides, and the production of collagen among the fibrous proteins.

Briefly, the acidic mucopolysaccharide production promoting agent according to the present invention particularly serves as "a hyaluronic acid production promoting agent," and the fibrous protein production promoting agent according to the present invention particularly serves as "a collagen production promoting agent."

### 1. Production promoting agent for acidic mucopolysaccharides such as hyaluronic acid

Hyaluronic acid has a variety of functions, to include maintaining water in intercellular space; sustaining cells by forming a jelly-like matrix in tissue; maintaining lubricity and softness of tissues; resisting an external force such as that which can mechanically induce damage; and preventing bacterial infection (*BIO INDUSTRY,* Vol. 8, p. 346, 1991). The hyaluronic acid content of skin decreases with age, to possibly cause skin aging such as wrinkles, fine wrinkles, or dryness. Thus, there have been proposed cosmetics formulated with collagen or hyaluronic acid serving as skin-normalizing agents. However, these cosmetics are effective only for moisturizing the skin surface and are essentially unable to normalize aged skin.

The problems involved in conventional art can be resolved through the agent according to the present invention, which promotes the production of hyaluronic acid, an acidic mucopolysaccharide, from sites such as fibroblasts and epidermal cells, to thereby internally activate the skin.

When the agent according to the present invention is used in order to promote the production of in particular acidic mucopolysaccharides such as hyaluronic acid and the above-described extract is used as an active ingredient, no particular limitation is imposed on the amount of the extract incorporated into the agent according to the present invention, and it may be dependent on the extraction material and the form of the agent.

For example, when the extract of the root of Mishima Saiko is used as an active ingredient of the agent according to the present invention, the amount of the extract is preferably selected from 0.00001-10.0 wt.% as reduced to the solid content, based on the total amount of the agent, particularly preferably 0.00005-5.0 wt.%.

When the agent according to the present invention is used in order to promote the production of in particular acidic mucopolysaccharides such as hyaluronic acid and the above-described saikosaponin is used as an active ingredient, no definite limitation is imposed on the amount of saikosaponin incorporated into the agent according to the present invention, and it may be dependent on the type of saikosaponin and the specific form of the agent. However, the amount is preferably selected from approximately 0.000001-10.0 wt.% based on the total amount of the agent, particularly preferably 0.00001-1.0 wt.%.

When the agent according to the present invention is used in order to promote the production of in particular acidic mucopolysaccharides such as hyaluronic acid and the above-described saikogenin is used as an active ingredient, no definite limitation is imposed on the amount of saikogenin incorporated into the agent according to the present invention, and it may be dependent on the type of saikogenin and the specific form of the agent. However, the amount is preferably selected from approximately 0.000001-10.0 wt.% based on the total amount of the agent, particularly preferably 0.00001-1.0 wt.%.

Although above-described saikosaponin has already been known to exhibit a skin activation effect (See Japanese Patent Publication (*kokoku*) No. 4-36137), the skin activation effect is limited to a promotion effect for proliferating fibroblasts of human skin; a promotion effect for healing a wound of a rat, which is derived from the promotion effect for proliferating fibroblasts; and an effect for normalizing rough skin tested by use of an actual human subject. No specific mechanism has been known, other than that of the promotion effect for proliferating fibroblasts.

As a matter of course, saikosaponin has never been known to exhibit a promotion effect for producing acidic mucopolysaccharides such as hyaluronic acid, nor has it been known to exhibit a promotion effect for producing extracellular matrix.

### 2. Production promoting agent for fibrous proteins such as collagen

The skin has a structure comprising the corneum, epidermis, basement membrane, and dermis. A fibrous structure which lies between the basement membrane and the dermis and can be observed by use of an electron microscope is formed of what are called "anchoring fibers." Ends of some groups of anchoring fibers enter the lamina densa of the basement membrane, whereas the opposite ends thereof enter an "anchoring plaque" formed of substances including type-IV collagen and laminin. Other groups of anchoring fibers enter the dermis more deeply and can form a giant network structure by aggregating with anchoring plaques. Such a network structure is considered to play an important role in binding between the epidermis and the dermis (Keene, *J. Cell. Biol*., 104:611-621, 1987).

Type-VII collagen is known to be the predominant component of anchoring fibers. It is assumed that the collagen molecule comprises three α1-chains having a molecular weight of about 300 KDa; dimers thereof are formed via intermolecular disulfide bonds; and the dimers further associate to form fibers. Therefore, type-VII collagen apparently plays a very important role in binding the epidermis and the dermis. In a culture system, epidermal cells and fibroblasts form type-VII collagen in a very small amount. However, the mechanism whereby the *in vivo* metabolism of type-VII collagen is controlled remains unclear.

One key to elucidating the role of anchoring fibers is "congenital epidermal hydroa," which is apparently caused through mutation of type-VII collagen. In this disease, formation of anchoring fibers is impossible due to genetic mutation of type-VII collagen, to thereby fail to effect binding between the epidermal layer and the dermal layer. Thus, the proper skin structure is not maintained (Christiano, *Nature Genet*. 4:62-66, 1993).

Regarding normal skin, the number of anchoring fibers is reported to decrease with the progress of physiological or light-induced aging of normal skin (Takuo TSUJI, *Nippi Kaishi*, 105:963-975, 1995; Tidman and Eady, *J. Invest. Dermatol.*, 83:448-453, 1984). It has been also reported that, with aging of the donor, the production of type-VII collagen a predominant component of anchoring fibers from fibroblasts derived from the skin of the donor is reduced both on a protein level and on an mRNA level in an *in vitro* culture system (Chen, *J. Invest. Dermatol*., 102; 205-209, 1994).

Although application of retinoic acid is a well-known technique for normalizing age-developing phenomena such as wrinkles, it has been also reported that the number of anchoring fibers significantly increases by continuous application of retinoic acid to UV-damaged skin. This result indicates that restoration of the number of anchoring fibers relates to normalization of wrinkles (Woodley, *JAMA*, 263:3057-3059, 1990).

Thus, in order to maintain young-looking skin, the importance for maintaining the number and quality of the anchoring fibers is again recognized, as the relationship between anchoring fibers and skin aging becomes clear.

The agent according to the present invention promotes the production of collagen as a fibrous protein from fibroblasts and epidermal cells, to thereby enhance the number and quality of anchoring fibers; activate extracellular matrix; normalize skin tissue; strengthen the binding between the epidermis and the dermis; prevent wrinkles and fine wrinkles; restore youthful skin structure; and prevent skin aging.

When the agent according to the present invention is used in order to promote the production of in particular fibrous proteins such as collagen and the above-described extract is used as an active ingredient, no particular limitation is imposed on the amount of the extract incorporated into the agent according to the present invention, and it may be dependent on the extraction material and the form of the agent.

For example, when the extract of the root of Mishima Saiko is used as an active ingredient of the agent according to the present invention, the amount of the extract is preferably selected from 0.00001-10.0 wt.% as reduced to the solid content, based on the total amount of the agent, particularly preferably 0.00005-5.0 wt.%.

When the agent according to the present invention is used in order to promote the production of in particular fibrous proteins such as collagen and the above-described saikosaponin is used as an active ingredient, no definite limitation is imposed on the amount of saikosaponin incorporated into the agent according to the present invention, and it may be dependent on the type of saikosaponin and the specific form of the agent. However, the amount is preferably selected from approximately 0.000001-10.0 wt.% based on the total amount of the agent, particularly preferably 0.00001-1.0 wt.%.

When the agent according to the present invention is used in order to promote the production of in particular fibrous protein such as collagen and the above-described saikogenin is used as an active ingredient, no definite limitation is imposed on the amount of saikogenin incorporated into the agent according to the present invention, and it may be dependent on the type of saikogenin and the specific form of the agent. However, the amount is preferably selected from approximately 0.000001-10.0 wt.% based on the total amount of the agent, particularly preferably 0.00001-1.0 wt.%.

Although, as described above, saikosaponin has already been known to exhibit an skin activation effect (See Japanese Patent Publication (*kokoku*) No. 4-36137), the skin activation effect is limited to a promotion effect for proliferating fibroblasts of human skin; a promotion effect for healing a wound of a rat, which is derived from the promotion effect for proliferating fibroblasts; and an effect for normalizing rough skin tested by use of an actual human subject. No specific mechanism has been known, other than that of the promotion effect for proliferating fibroblasts.

As a matter of course, saikosaponin has never been known to exhibit a promotion effect for producing fibrous proteins such as collagen.

The agent of the present invention may take various forms including aqueous solutions, oily liquids, other types of solutions, emulsions, creams, gels, suspensions, micro-capsules, powders, granules, capsules, and solid products. After preparation into these forms by any known method, the agents may be further processed into products including lotions, milks, creams, ointments, plasters, cataplasms, aerosols, water-oil two-layered systems, water-oil-powder three-layered systems, injections, internal use forms (such as tablets, powders, granules, pills, syrups, and troches), and suppositories, and may be applied to the body by way of spreading, patches, spraying, injection, oral ingestion, or insertion. Of the mentioned product forms, a suitable form which meets the object of the present invention is a skin preparation for external use, which includes lotions, milks, creams, ointments, plasters, cataplasms, and aerosols. As used herein, "a skin preparation for external use" refers to drugs, quasi-drugs such as ointments, and cosmetics (including basic cosmetics such as face cleansers, milky lotions, creams, gels, essences (beauty lotions), packs and masks; make-up cosmetics such as foundations and lipsticks; oral cavity cosmetics; fragrances; hair cosmetics; and body cosmetics).

Into the agent of the present invention which may take any of the above forms and product forms, in accordance with the forms and product forms of interest and needs, there may be incorporated the following ingredients, which include generally known excipients and perfumes, fats and oils, higher fatty acids, higher alcohols, synthetic ester oils, silicones, surfactants, preservatives, sequestering agents, water-soluble polymers, thickeners, powder components (such as pigments), ultraviolet (UV) shielding agents, moisturizers, antioxidants, pH regulators, detergents, desiccants, and emulsifiers. Pharmaceutically active ingredients may further be incorporated into the agent of the present invention so long as they do not impede the intended effect.

Examples of fats and oils include liquid fats and oils, solid fats and oils, waxes, and oily components, wherein liquid fats and oils include avocado oil, tsubaki oil, evening primrose oil, turtle oil, macademia nut oil, corn oil, mink oil, olive oil, rape seed oil, egg yolk oil, sesame oil, persic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerin, glycerin trioctanoate, and glycerin triisopalmitate; solid fats and oils include cacao butter, coconut oil, horse fat, hydrogenated coconut oil, palm oil, beef tallow, mutton fallow, hydrogenated beef tallow, palm kernel oil, lard, beef bone fat, Japan wax kernel oil, hydrogenated oil, beef foot oil, Japan wax, and hydrogenated castor oil; waxes include beeswax, candelilla wax, cotton wax, carnauba wax, insect wax, spermaceti, montan wax, rice bran wax, lanolin, kapok wax, lanolin acetate, liquid lanolin, sugar cane wax, a lanolin fatty acid isopropyl ester, hexyl laurate, hydrogenated lanolin, jojoba wax, hard lanolin, shellac wax, polyoxyethylene (hereinafter referred to as POE) lanolin alcohol ether, POE lanolin alcohol acetate, POE cholesterol ether, lanolin fatty acid polyethylene glycol, and POE hydrogenated lanolin alcohol ether; and oily components include liquid paraffin, ozocerite, squalene, pristane, paraffin, ceresine, squalane, vaseline, and microcrystalline wax.

Examples of higher fatty acids include lauric acid, myristic acid, palmitic acid, stearic acid, behenic acid, oleic acid, 12-hydroxystearic acid, undecylenoic acid, tol acid, isostearic acid, linoleic acid, linolenic acid, eicosapentaenoic acid (EPA), and docosahexaenoic acid (DHA).

Examples of higher alcohols include linear alcohols such as lauryl alcohol, cetyl alcohol, stearyl alcohol, behenyl alcohol, myristyl alcohol, oleyl alcohol, and cetostearyl alcohol, and branched alcohols such as monostearyl glycerin ether (batyl alcohol), 2-decyltetradecynol, lanolin alcohol, cholesterol phytosterol, hexyldodecanol, isostearyl alcohol, and octyldodecanol.

Examples of synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid ester, N-alkyleneglycol monoisostearate, neopentyl glycol dicaprate, diisostearyl malate, glycerin di-2-heptylundecanoate, trimethylolpropane tr-2-ethylhexylate, trimethylolpropane triisostearate, pentaerythritol tetra-2-ethylhexylate, glycerin tri-2-ethylhexylate, trimethylolpropane triisostearate, cetyl 2-ethylhexanoate, 2-ethylhexyl palmitate, glycerin trimyristate, glycerin tri-2-heptylundecanoate, methyl castor oil fatty acid, oleyl oleate, cetostearyl alcohol, acetoglyceride, 2-heptylundecyl palmitate, diisobutyl adipate, an N-lauroyl-L-glutamic acid 2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, and triethyl citrate.

Examples of silicones include linear polysiloxanes such as dimethylpolysiloxane, methylphenyl polysiloxane, and methyl hydrogen polysiloxane; cyclic polysiloxanes such as decamethylpolysiloxane, dodecamethylpolysiloxane, and tetramethyl tetrahydrogen polysiloxane; and silicone resins and silicone rubbers forming three-dimensional network structures.

The agent of the present invention may contain any type of surfactant; i.e., an anionic surfactant, a cationic surfactant, an amphoteric surfactant, or a nonionic surfactant.

Examples of anionic surfactants include components for soap; fatty acid soap such as sodium laurate or sodium palmitate; higher alkyl sulfates such as sodium lauryl sulfate and potassium lauryl sulfate; alkyl ether sulfates such as triethanolamine POE laurylether sulfate and sodium POE laurylether sulfate; N-acylsarcosinate such as sodium lauroylsarcosinate; higher fatty acid amide sulfonates such as an sodium-N-myristoyl N-methyl taurate, sodium-N-cocoyl N-methyl taurate, and sodium-N-lauryl N-methyl taurate; phosphates such as sodium POE oleylether phosphate and POE stearylether phosphate; sulfosuccinates such as di-2-ethylhexyl sodium sulfosuccinate, sodium monolauroyl monoethanolamide polyoxyethylene sulfosuccinate, and sodium lauryl polypropylene glycol sulfosuccinate; alkylbenzenesulfonic acids or alkylbenzenesulfonates such as linear sodium dodecylbenzenesulfonate, linear triethanolamine dodecylbenzenesulfonate, and linear dodecylbenzenesulfonic acid; N-acylglutamates such as monosodium N-lauroyl glutamate, disodium N-stearoyl glutamate, and monosodium N-myristoyl-L-glutamate; higher fatty acid ester sulfate such as sodium hydrogenated glyceryl cocoate sulfate; sulfonated oil such as Turkey red oil; POE alkyl ether carboxylate; POE alkylally ether carboxylate; α-olefin sulfonate; higher fatty acid ester sulfonate; secondary alcohol sulfate; higher fatty acid alkylolamide sulfate; sodium lauroyl monoethanolamide succinate; di-triethanolamine N-palmitoylaspartate; and sodium caseinate.

Examples of cationic surfactants include alkyltrimethylammonium salts such as stearyltrimethylammonium chloride and lauryltrimethylammonium chloride; dialkyldimethylammonium salt such as distearyldimethylammonium chloride; alkylpyridinium salts such as poly(N,N*'*-dimethyl-3,5-methylenepiperidinium) chloride and cetylpyridinium chloride; alkyl quaternary ammonium salts; alkyldimethylbenzylammonium salts; alkylisoquinolinium salts; dialkylmorpholinium salts; POE alkylamines; alkylamine salts; polyamine fatty acid derivatives; amyl alcohol fatty acid derivatives; benzalkonium chloride; and benzethonium chloride.

Examples of amphoteric surfactants include imidazoline amphoteric surfactants such as a 2-undecyl-N,N,N-(hydroxyethylcarboxymethyl)-2-imidazoline sodium salt and a 2-cocoyl-2-imidazoliniumhydroxide-1-carboxyethyloxy 2 sodium salt; betaine amphoteric surfactants such as 2-heptadecyl-N-carboxymethyl-N-hydroxyethylimidazolinium betaine, lauryldimethylaminoacetic acid betaine, alkylbetaine, amidebetaine, and sulfobetaine.

Examples of oleophilic nonionic surfactants include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerolsorbitan penta-2-ethylhexylate, and diglycerolsorbitan tetra-2-ethylhexylate; mono- or polyglyceride such as cotton seed oil fatty acid monoglyceride, glyceryl monoerucate, glyceryl sesquioleate, glyceryl monostearate, glyceryl α,α*'*-oleate pyroglutamate, and glyceryl monostearate malate; propylene glycol fatty acid esters such as propylene glycol monostearate; hydrogenated castor oil derivatives; glycerin alkylethers; and polyoxyethylene methylpolysiloxane copolymers.

Examples of hydrophilic nonionic surfactants include POE sorbitan fatty acid esters such as POE sorbitan monooleate, POE sorbitan monostearate, POE sorbitan monooleate, and POE sorbitan tetraoleate; POE sorbitol fatty acid esters such as POE sorbitol monolaurate, POE sorbitol monooleate, POE sorbitol pentaoleate, and POE sorbitol monostearate; POE glycerin fatty acid esters such as POE glycerin monostearate, POE glycerin monoisostearate, and POE glycerin triisostearate; POE fatty acid esters such as POE monooleate, POE distearate, POE monodioleate, and ethylene glycol distearate; POE alkyl ethers such as POE lauryl ether, POE oleyl ether, POE stearyl ether, POE behenyl ether, POE 2-octyldodecyl ether, and POE cholestanol ether; POE alkylphenyl ethers such as POE octylphenyl ether, POE nonylphenyl ether, and POE dinonylphenyl ether; pluaronic such as Pluronic®; POE·polyoxypropylene (hereinafter polyoxypropylene will be referred to as POP) alkyl ethers such as POE·POP cetyl ether, POE·POP 2-decyltetradecyl ether, POE·POP monobutyl ether, POE·POP hydrogenated lanolin, and POE·POP glycerin ether; tetra POE·tetra POP ethylenediamine condensation products such as Tetronic®; POE castor oil or POE hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearate, and POE hydrogenated castor oil maleate; POE beeswax·lanolin derivative such as POE sorbitol beeswax; alkanol amides such as coconut oil fatty acid diethanolamide, lauric acid monoethanolamide, and fatty acid isopropanolamide; POE propylene glycol fatty acid esters; POE alkylamines; POE fatty acid amides; sucrose fatty acid esters; POE nonylphenyl formaldehyde condensation products; alkylethoxydimethylamine oxides; and trioleylphosphoric acid.

Examples of preservatives include methyl paraben, ethyl paraben, and butyl paraben.

Examples of sequestering agents include EDTA, alkali metal salts of edetate such as disodium edetate, and alkali earth metal salts of edetate such as calcium edetate.

Examples of water-soluble polymers include natural polymers, semi-synthetic polymers, synthetic polymers, and inorganic polymers.

Examples of natural water-soluble polymers include vegetable polymers such as gum arabic, tragacanth gum, galactan, carob gum, guar gum, gum karaya, carageenan, pectin, agar, quinceseed (quince), algae colloid (brown algae extracts), and starch (e.g., rice, corn, potato, and wheat); microbial-originating polymers such as dextran, succinoglucan, and pullulan; starch polymers such as carboxymethyl starch and methylhydroxypropyl starch; and animal-originating, polymers such as collagen, casein, albumin, and gelatin.

Examples of semi-synthetic water-soluble polymers include cellulose type polymers such as methylcellulose, nitrocellulose, ethylcellulose, hydroxypropyl methylcellulose, hydroxyethylcellulose, sodium cellulose sulfate, hydroxypropylcellulose, sodium carboxymethyl cellulose (CMC), crystalline cellulose, and cellulose powders; and alginate polymers such as sodium alginate and propylenglycol alginate.

Examples of synthetic water-soluble polymers include vinyl polymers such as polyvinyl alcohol (PVA), poly (vinylmethyl) ether (PVM), and carboxyvinylpolymer (CARBOPOL, etc); polyoxyethylene polymers; polyoxyethylene polyoxypropylene copolymers; acrylic polymers such as sodium polyacrylate, poly(ethyl acrylate), and polyacrylamide; and examples of inorganic water-soluble polymers include polyethyleneimine, cationic polymers, bentonite, magnesium aluminum silicate, laponite, hectorite, and silicic anhydride.

The aforementioned water soluble polymers may also be used as thickeners. Thus, examples of thickeners include carageenan, gum karaya, tragacanth gum, carob gum, quinceseed (quince), casein, dextrin, gelatin, sodium pectinate, sodium alginate, methylcellulose, ethylcellulose, CMC, hydroxyethylcellulose, hydroxypropylcellulose, PVA, PVM, polyvinylpyrolidone (PVP), sodium polyacrylate, carboxyvinylpolymer, locust bean gum, guar gum, tamarind gum, dialkyldimethylammonium cellulose sulfate, xanthan gum, magnesium aluminum silicate, bentonite, and hectorite.

Examples of powder components include:
inorganic powders such as talc, kaolin, mica, sericite, muscovite, phlogopite, lepidolite, biotite, lithia mica, vermiculite, magnesium carbonate, strontium silicate, barium silicate, calcium silicate, magnesium silicate, metal salts of tungstate, magnesium, silica, zeolite, barium sulfate, calcined calcium sulfate (calcined plaster), calcium phosphate, fluorapatite, hydroxyapatite, ceramic powders, metallic soaps (e.g., zinc myristate, calcium palmitate, and aluminum stearate), and boron nitride;
organic powders such as polyamide resin powders (e.g., nylon powders), polyethylene powders, polymethyl methacrylate powders, polystyrene powders, styrene-acrylic acid copolymer resin powders, benzoguanamine resin powders, polytetrafluoroethylene powders, and cellulose powders;
inorganic white pigments (e.g., titanium dioxide and zinc oxide), inorganic red pigments (e.g., iron oxide (red iron oxide) and iron titanate), inorganic brown pigments (e.g., γ-iron oxide), inorganic yellow pigments (e.g., yellow iron oxide and loess), inorganic black pigments (e.g., black iron oxide, carbon black, and lower titanium oxide), inorganic violet pigments (e.g., ammonium manganese pyrophosphate (manganese violet) and cobalt violet), inorganic green pigments (e.g., chromium oxide, chromium hydroxide, and cobalt titanate), inorganic blue pigments (e.g., ultramarine and prussian blue), pearl pigments (e.g., titanium oxide coated mica, titanium oxide coated oxybismuth, titanium oxide coated talc, colored-titanium-oxide-coated mica, bismuth oxychloride, and fish scales), and metallic powdery pigments (e.g., aluminum powders and copper powders); and
colorants such as organic pigments (e.g., Red #201, Red #202, Red #204, Red #205, Red #220, Red #226, Red #228, Red #405, Orange #203, Orange #204, Yellow #205, Yellow #401, and Blue #404), organic lake pigments of zirconium, barium, aluminum, etc. (e.g., Red #3, Red #106, Red #227, Red #230, Red #401, Red #505, Orange #205, Yellow #4, Yellow #5, Yellow #202, Yellow #203, Green #3, and Blue #1), natural pigments (e.g., chlorophyll and β-carotene), titanium yellow, carthamine, and safflower red.

Examples of UV shielding agents (whose concept encompasses both "UV absorbing agents" which absorb UV rays chemically, and "UV blocking agents" which scatter or reflect UV rays through physical actions) include p-aminobenzoic acid UV absorbing agents such as p-aminobenzoic acid; anthranilic acid UV absorbing agents such as methyl anthranilate; salicylic acid UV absorbing agents such as octyl salicylate, phenyl salicylate, and homomenthyl salicylate; cinnamic acid UV absorbing agents such as isopropyl p-methoxycinnamate, octyl p-methoxycinnamate, 2-ethylhexyl p-methoxycinnamate, glyceryl di-p-methoxycinnamate mono-2-ethylhexanate, and [4-bis(trimethylsiloxy)methylsilyl-3-methylbutyl]-3,4,5-tri-methoxycinnamate; benzophenone UV absorbing agents such as 2,4-dihydroxybenzophenone, 2-hydroxy-4-methoxybenzophenone, 2-hydroxy-4-methoxybenzophenone-5-sulfonic acid, and sodium 2-hydroxy-4-methoxybenzophenone-5-sulfonate; and UV absorbing agents such as urocanic acid, ethyl urocanate, 2-phenyl-5-methylbenzoxazole, 2-(2'-hydroxy-5'-methylphenyl)benztriazole, and 4-tert-butyl-4'-methoxydibenzoylmethane.

Examples of UV shielding agents also include titanium oxide, talc, carmine, bentonite, kaolin, and zinc oxide. Some of these examples are also included in the examples of powder components.

Examples of moisturizers include polyethylene glycol, propylene glycol, dipropylene glycol, 1,3-butylene glycol, hexylene glycol, glycerol, diglycerol, xylitol, maltitol, maltose, D-mannitol, starch syrup, glucose, fructose, lactose, sodium chondroitinsulfate, sodium hyaluronate, sodium adenosine phosphate, sodium lactate, bile acid salts, pyrrolidonecarboxylic acid, glucosamine, and cyclodextrin.

Examples of active ingredients include, as vitamins, vitamin A such as vitamin A oil, retinol, retionol acetate, or retinol palmitate; vitamin B₂ such as riboflavin, riboflavin lactate, or flavin adenine nucleotide; vitamin B₆ such as pyridoxine hydrochloride or pyridoxine dioctanoate; vitamin C such as L-ascorbic acid, L-ascorbic acid monopalmitate, L-ascorbic acid dipalmitate, sodium L-ascorbic acid-2-sulfate, L-ascorbic acid phosphate, a DL-α-tocopherol-L-ascorbic acid phosphoric acid diester dipotassium salt, L-ascorbic stearate, or L-ascorbic acid-2-glucoside; pantothenic acids such as calcium pantothenate, D-pantothenyl alcohol, panthothenyl ethyl ether, and acetylpantothenyl ethyl ether; vitamin D such as vitamin D₂ (ergocalciferol) or cholecalciferol; vitamin E such as α-tocopherol, tocopherol acetate, DL-α-tocopheryl nicotinate, or DL-α-tocopheryl succinate; vitamin P, and biotin.

Examples of active ingredients also include whitening agents such as placenta extracts, glutathione, saxifrage extracts, and arbutin; skin activators such as royal jelly, photosensitizing dye #401, and calf blood extracts; blood circulation promoters such as 4-hydroxy-3-methoxybenzyl nonylic acid, benzyl nicotinate, β-butoxyethyl nicotinate, capsaicin, zingerone, cantharis tincture, ichthammol, caffeine, tannic acid, α-borneol, inositol hexanicotinate, cyclandelate, cinnarizine, tolazoline, acetylcholine, verapamil, cephalanthin, and γ-oryzanol; anti fat leaking agents such as sulfur and thianthol; antiphlogistic agents such as glycyrrhizinic acid derivatives, glycyrrhetinic acid derivatives, azulene, salicylic acid derivatives, hinokitiol, zinc oxide, and allantoine; female sex hormone agents such as estradiol and ethinylestradiol; anti-inflammatory agents such as amino acids (e.g., arginine, aspartic acid, cystine, cysteine, methionine, serine, leucine, and tryptophan); astringents such as tannic acid and citric acid; refrigerants such as L-menthol and camphor; and lysozyme chloride.

Various extracts which have variety of effects may also be incorporated into the agent of the present invention. Examples of the extracts include grape extracts, *Houttuynia* extracts, mallow extracts, loquat extracts, phellodendron bark extracts, coix extracts, swertia herb extracts, saffron extracts, cnidium rhizome extracts, iris extracts, Japanese coptis extracts, sweet clover extracts, birch extracts, lily extracts, white nettle extracts, glycyrrhiza extracts, peony root extracts, saponaria extracts, restharrow extracts, sponge gourd extracts, capsicum extracts, cinchona extracts, Japanese angelica root extracts, citrus unshiu extracts, saxifrage extracts, sophora root extracts, nuphar extracts, fennel extracts, Primula Veris extracts, Primula Vulgaris extracts, rose extracts, rehmannia root extracts, lemon extracts, garlic extracts, Belamcanda Chinensis extracts, lithospermum root extracts, aloe extracts, calamus rhizome extracts, eucalyptus extracts, rosemary extracts, horsetail extracts, sage extracts, thyme extracts, green tea extracts, seaweed extracts, cucumber extracts, clove extracts, raspberry extracts, balm mint extracts, ginseng extracts, horse chestnut extracts, peach extracts, peach leaf extracts, mulberry bark extracts, cornflower extracts, witch hazel extracts, ginger extracts, hestnut rose extracts, placental extracts, thymus extracts, and silk extracts.

The production of hyaluronic acid is promoted synergistically when a female sex hormone agent (such as estradiol or ethinylestradiol) or a blackberry lily extract is incorporated into the agent of the present invention.

The production of collagen is promoted synergistically when vitamin C is incorporated into the agent of the present invention. Examples of the vitamin C includes such as L-ascorbic acid, L-ascorbic acid monopalmitate, L-ascorbic acid dipalmitate, sodium L-ascorbic acid-2-sulfate, L-ascorbic acid phosphate, a DL-α-tocopherol-L-ascorbic acid phosphoric acid diester dipotassium salt, L-ascorbic stearate, or L-ascorbic acid-2-glucoside.

### Examples

The present invention will next be described in detail by way of examples, which should not be construed as limiting the invention thereto.

### [Reference Example] Preparation of specimens

### (1) Preparation of test specimens

Dried root of Mishima Saiko (500 g) was immersed in a 50% aqueous solution of 1,3-butylene glycol (10 times the amount of the dried root), and extraction was carried out at 70°C for one day. The resultant mixture was allowed to stand in a dark and cool place for aging, and was then filtered to thereby obtain an extract of the dried root (hereinafter referred to as "Saiko extract") (4700 g).

The filtrate obtained in the above step was passed through a column of a synthetic adsorbent (Dia-ion HP-2, product of Mitsubishi Chemical Co., Ltd.). Subsequently, a 40% ferric chloride solution was added to the resultant solution to thereby convert saikosaponin a and saikosaponin d to saikosaponin b1 and saikosaponin b2, respectively.

The saikosaponins were eluted by use of ethanol, and the eluted solution was concentrated under reduced pressure and evaporated to dryness. The dry product was dissolved in methanol, and the solution was subjected to fractionation treatment by use of a silica gel column and high performance liquid chromatography treatment, to thereby obtain salkosaponin b1 (hereinafter also referred to as "SSb1").

The corresponding saikogenin was obtained via saccharide-removal reaction of the thus-obtained saikosaponin, through the above-described generally known method.

### [Test Example A] Hyaluronic acid production test and actual application test

### (1) Evaluation of action of specimens on human epidermal cells for producing hyaluronic acid

The action of the Saiko extract and SSb1 on human epidermal cells for producing hyaluronic acid was evaluated by use of human foreskin-originating epidermal cells (product of Kurabo Industries Ltd., hereinafter referred to as "epidermal cells"). Specifically, epidermal cells (5 × 10⁴ cells/well) suspended in KGM medium (product of Kurabo Industries Ltd., hereinafter referred to as a "culture medium for epidermal cells") were placed in a 24-well culture plate (product of Falcon Co.) and cultured to a confluent state (culturing conditions: 37°C, 5% CO₂). Subsequently, the culture medium was exchanged for a culture medium for epidermal cells containing Saiko extract (10.0 µg/ml) or SSb1 (0.1 µg/ml), or a culture medium for epidermal cells without any specimen for control, and the epidermal cells were cultured for 48 hours under the same conditions. After 48 hours, the culture supernatant was collected and the amount of hyaluronic acid was measured. The measurement was carried out by use of a measuring kit, Hyaluronic Aacid Plate "Chugai" (product of Chugai Pharmaceutical Co., Ltd.).

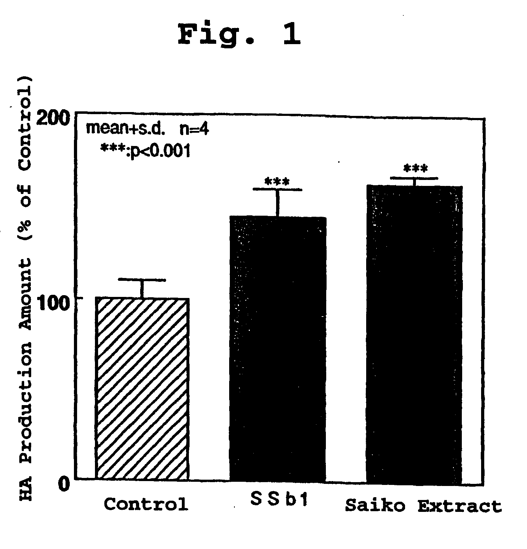

The result are shown in Fig. 1. The epidermal cells cultured in the medium containing Saiko extract and those cultured in the medium containing SSb1 produced more hyaluronic acid than did the epidermal cells cultured with the medium without any specimen (control).

Therefore, Saiko extract and SSb1 are proved to have action for promoting the production of hyaluronic acid from epidermal cells.

### (2) Evaluation of action of specimens on human skin fibroblasts for producing hyaluronic acid

The action of the Saiko extract and SSb1 on fibroblasts for producing hyaluronic acid was evaluated by use of human skin-originating fibroblasts (obtained by treating surgically obtained skin through an explant method, hereinafter referred to as "fibroblasts"). Specifically, fibroblasts (1 × 10⁴ cells/well) suspended in a 1%FBS/DMEM medium (FBS medium: product of GIBCO Ltd., DMEM medium: product of Nissui Pharmaceutical Co., Ltd.) were placed in a 24-well culture plate (product of Falcon Co.) and cultured to a confluent state (culturing conditions: 37°C, 5% CO₂). Subsequently, the culture medium was exchanged for a culture medium for epidermal cells containing Saiko extract (10.0 µg/ml) or SSb1 (0.1 µg/ml), or a culture medium for epidermal cells without any specimen for control, and the epidermal cells were cultured for 48 hours under the same conditions.

After 48 hours, the culture supernatant was collected, and the amount of hyaluronic acid was measured. The measurement was carried out by use of a measuring kit, Hyaluronic acid plate "Chugai" (product of Chugai Pharmaceutical Co., Ltd.).

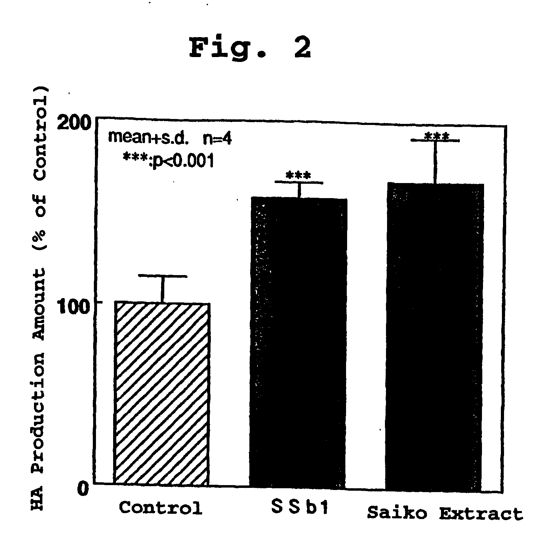

The results are shown in Fig. 2. The fibroblasts cultured in the medium containing Saiko extract and those cultured in the medium containing SSb1 produced a greater amount of hyaluronic acid than did the fibroblasts cultured in the medium without any specimen (control).

Therefore, Saiko extract and SSb1 are proved to have action for promoting the production of hyaluronic acid from human fibroblasts.

### (3) Actual application test

Agents having formulations shown in Table 1A which were obtained in Examples 1A and 2A and Comparative Example 1A were tested for actual application as described below. The amounts are based on wt.%.

**Table 1A**

| | | Ex. 1A | Ex. 2A | Comp. Ex. 1A |
|---|---|---|---|---|
| (1) | Cetostearyl alcohol | 3.5 | 3.5 | 3.5 |
| (2) | Squalane | 40.0 | 40.0 | 40.0 |
| (3) | Beeswax | 3.0 | 3.0 | 3.0 |
| (4) | Hydrogenated lanolin | 4.0 | 4.0 | 4.0 |
| (5) | Ethylparaben | 0.3 | 0.3 | 0.3 |
| (6) | Polyoxyethylene (20) sorbitan esters of monopalmitic acid | 2.0 | 2.0 | 2.0 |
| (7) | Monoglycerol stearate | 3.0 | 3.0 | 3.0 |
| (8) | Saiko extract | 3.0 | -- | -- |
| (9) | Saikosaponin b1 | -- | 0.05 | -- |
| (10) | Sodium N-stearoylglutamate | 0.5 | 0.5 | 0.5 |
| (11) | Perfume | 0.03 | 0.03 | 0.03 |
| (12) | 1,3-Butylene glycol | 5.0 | 5.0 | 5.0 |
| (13) | Polyethylene glycol 1500 | 5.0 | 5.0 | 5.0 |
| (14) | Purified water | bal. | bal. | bal. |

〈Process〉 Ingredients (1) through (11) were dissolved by heating to thereby prepare an oily phase, whereas ingredients (12) and (13) were dissolved in purified water (14) and the resultant solution was maintained at 70°C to thereby prepare an aqueous phase. The aqueous phase was added into the oily phase with stirring. The product was subjected to a homo-mixer treatment to yield minute, emulsified particles, and quenched with stirring to thereby obtain a target cream.

In the actual application test, healthy adult women selected at random and between the ages 30 and 71 inclusive served as test subjects. The agents of Examples and Comparative Example were each used for four continuous weeks by a group consisting of 30 subjects. The following effects were evaluated as average ratings of the 30 subjects: (1) effect for improving skin moistness or moisture; (2) effect for improving tension and sagging of the skin; and (3) effect for improving wrinkles and fine wrinkles.

### (1) Ratings of effect for improving skin moisture

| | |
|---|---|
| Greatly improved | 3 |
| Improved | 2 |
| Somewhat improved | 1 |
| No effect | 0 |
| Deteriorated | -1 |

### (2) Ratings of effect for improving tension and sagging of the skin

| | |
|---|---|
| Greatly improved | 3 |
| Improved | 2 |
| Somewhat improved | 1 |
| No effect | 0 |
| Deteriorated | -1 |

### (3) Ratings of effect for improving wrinkles and fine wrinkles

| | |
|---|---|
| Greatly improved | 3 |
| Improved | 2 |
| Somewhat improved | 1 |
| No effect | 0 |
| Deteriorated | -1 |

The test results are shown in Table 2A.

**Table 2A**

| | Moisture | Tension/Sagging | Wrinkles/Fine wrinkles |
|---|---|---|---|
| Ex. 1A | 2.8 | 2.7 | 2.3 |
| Ex. 2A | 2.5 | 2.4 | 2.0 |
| Comp. Ex. 1A | 0.7 | 0.6 | 0.5 |

The results indicate that the groups using the agents of Examples 1A and 2A exhibit excellent improvement effect as compared to the group using the agent of Comparative Example 1A.

These three tests prove that the agent according to the present invention promotes the production of hyaluronic acid, which is a component of extracellular matrix, to thereby activate extracellular matrix; normalize skin tissue; and prevent skin aging.

### [Test Example B] Type-VII collagen production test and actual application test

### (1) Evaluation of action of specimens on human epidermal cells for producing type-VII collagen

The action of the Saiko extract and SSb1 on human epidermal cells for producing type-VII collagen was evaluated by use of human foreskin-originating epidermal cells (product of Kurabo Industries Ltd., hereinafter referred to as "epidermal cells"). Specifically, epidermal cells (5 × 10³ cells/well) suspended in KGM medium (product of Kurabo Industries Ltd., hereinafter referred to as a "culture medium for epidermal cells") were placed in a slide chamber (product of Nunc Co.). Subsequently, the culture medium was exchanged for a culture medium for expidermal cells containing Saiko extract (10.0 µg/ml) or SSb1 (0.1 µg/ml), or a culture medium for expidermal cells without any specimen for control, and the epidermal cells were cultured for 48 hours under the conditions of 37°C and 5% CO₂.

After removal of the culture medium from the system, the epidermal cells were fixed with paraformaldehyde. The fixed cells were caused to react with a monochronal antibody to type-VII collagen (Sakai, L. Y., *et al., J. Cell. Biol*., 103, 1577-1586, 1986) and an FITC labeled secondary antibody (product of Sigma Co.), and observed under a fluorescence microscope and photographed.

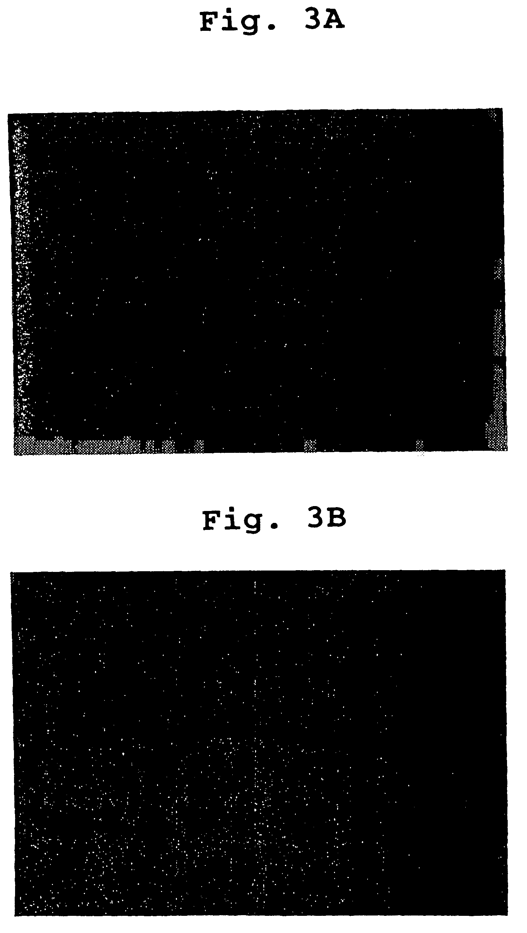

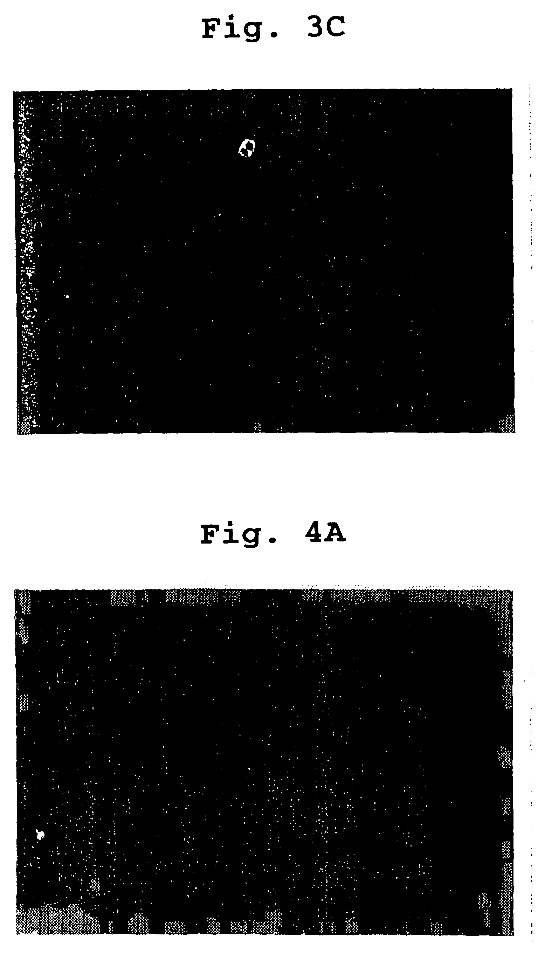

The results are shown in Figs. 3A (control), 3B (system containing SSb1), and 3C (system containing Saiko extract) [Figs. 3A through 3C are photographs showing morphology of organisms]. These photographs reveal that a number of epidermal cells are fluorescence-labeled and that the epidermal cells cultured in the medium containing Saiko extract and those cultured in the medium containing SSb1 produce more type-VII collagen than do the epidermal cells cultured in the medium without any specimen (control).

### (2) Evaluation of action of specimens on human skin fibroblasts for producing type-VII collagen

The action of the Saiko extract and SSb1 on fibroblasts for producing type-VII collagen was evaluated by use of human skin-originating fibroblasts (obtained by treating surgically-obtained skin through an explant method, hereinafter referred to as "fibroblasts"). Specifically, fibroblasts (5 × 10³ cells/well) suspended in a 1% FBS/DMEM medium (FBS medium: product of GIBCO Ltd., DMEM medium: product of Nissui Pharmaceutical Co., Ltd., hereinafter referred to as a "culture medium for fibroblasts") were placed in the above-described slide chamber. Subsequently, the culture medium was exchanged for a culture medium for epidermal cells containing Saiko extract (231.5 µg/ml) or SSb1 (0.1 µg/ml), or a culture medium for epidermal cells without any specimen for control, and the fibroblasts were cultured for 48 hours under the conditions of 37°C and 5% CO₂.

After removal of the culture medium from the system, the fibroblasts were fixed with paraformaldehyde. The fixed cells were caused to react with a monochronal antibody to type-VII collagen (Sakai, L. Y., *et al., J. Cell. Biol.*, 103, 1577-1586, 1986) and an FITC labeled secondary antibody (product of Sigma Co.), and observed under a fluorescence microscope and photographed.

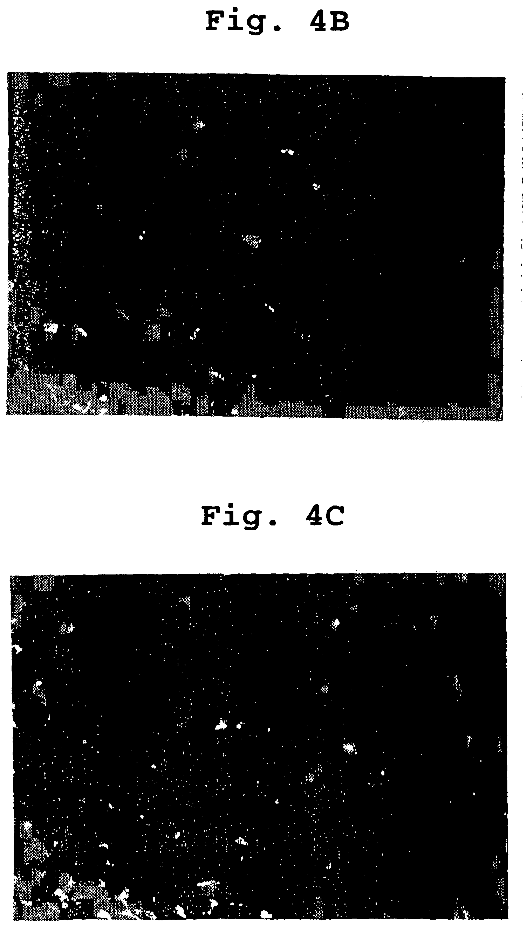

The results are shown in Figs. 4A (control), 4B (system containing SSb1), and 4C (system containing Saiko extract) [Figs. 4A through 4C are photographs showing morphology of organisms]. These photographs reveal that a number of epidermal cells are fluorescence-labeled and that the fibroblasts cultured with Saiko extract and those cultured with SSb1 produced a greater amount of type-VII collagen than did the fibroblasts cultured in the control medium.

### (3) Evaluation of action of specimens on in vitro ternary human skin model for producing type-VII collagen

The action of the Saiko extract and SSb1 on a ternary human skin model comprising human-originating fibroblasts and epidermal cells for producing type-VII collagen was evaluated.

Specifically, fibroblast (1 × 10⁵ cells/well)-suspended collagen solution (collagen; I-PA, product of Koken Ltd.) was prepared on ice, and collagen was gelled at 37°C. The gel was peeled off from a wall of the Petri dish and cultured in a culture medium for fibroblasts so as to shrink the collagen gel, to thereby prepare a dermis model.

Onto the dermis model, epidermal cells (2 × 10⁵ cells/well) were placed and cultured in a 5% FBS/DMEM-KGM medium (FBS medium: product of GIBCO Ltd:, DMEM medium: product of Nissui Pharmaceutical Co., Ltd., KGM medium: product of Kurabo Industries Ltd.) to thereby prepare a skin model. One week after preparation of the skin model, the culture medium was exchanged for a culture medium for epidermal cells containing Saiko extract (231.5 µg/ml) or SSb1 (0.1 µg/ml), a culture medium for epidermal cells without any specimen for control, and the epidermal cells were cultured for two weeks under the conditions of 37°C and 5% CO₂.

The skin model was embedded in liquid nitrogen to thereby prepare sections, which were caused to react with a monochronal antibody to type-VII collagen (Sakai, L. Y., *et al., J. Cell. Biol*., 103, 1577-1586, 1986) and an FITC labeled secondary antibody (product of Sigma Co.), and observed under a fluorescence microscope and photographed (photographs not shown in the description).

The results reveal that a number of epidermal cells are fluorescence-labeled and that the section cultured with Saiko extract and that cultured with SSb1 produced a greater amount of type-VII collagen than did the sections cultured in the control medium.

### (4) Actual application test

Agents having a formulation shown in Table 1B that were obtained in Examples 1B and 2B and Comparative Example 1B were tested for the actual application described below. The amounts are based on wt.%.

**Table 1B**

| | | Ex. 1B | Ex. 2B | Comp. Ex. 1B |
|---|---|---|---|---|
| (1) | Cetostearyl alcohol | 3.5 | 3.5 | 3.5 |
| (2) | Squalane | 40.0 | 40.0 | 40.0 |
| (3) | Beeswax | 3.0 | 3.0 | 3.0 |
| (4) | Hydrogenated lanolin | 4.0 | 4.0 | 4.0 |
| (5) | Ethylparaben | 0.3 | 0.3 | 0.3 |
| (6) | Polyoxyethylene (20) sorbitan ester of monopalmitic acid | 2.0 | 2.0 | 2.0 |
| (7) | Monoglycerol stearate | 3.0 | 3.0 | 3.0 |
| (8) | Saiko extract | 0.5 | -- | -- |
| (9) | Saikosaponin b1 | -- | 0.1 | -- |
| (10) | Sodium N-stearoylglutamate | 0.5 | 0.5 | 0.5 |
| (11) | Perfume | 0.03 | 0.03 | 0.03 |
| (12) | 1,3-Butylene glycol | 5.0 | 5.0 | 5.0 |
| (13) | Polyethylene glycol 1500 | 5.0 | 5.0 | 5.0 |
| (14) | Purified water | bal. | bal. | bal. |

〈Process〉 Ingredients (1) through (11) were dissolved by heating to thereby prepare an oily phase, whereas ingredients (12) and (13) were dissolved in purified water (14), and the resultant solution was maintained at 70°C to thereby prepare an aqueous phase. The aqueous phase was added into the oily phase with stirring. The product was subjected to a homomixer treatment to yield minute, emulsified particles, and quenched with stirring to thereby obtain a target cream.

In the actual application test, healthy adult women selected at random and between the ages of 36 and 69 inclusive served as test subjects. The agents of Examples and Comparative Example were each used for four continuous weeks by a group consisting of 30 subjects. The following effects were evaluated as average ratings of the 30 subjects: (1) effect for improving tension and sagging of the skin and (2) effect for improving wrinkles and fine wrinkles.

### (1) Ratings of effect for improving tension and sagging of the skin

| | |
|---|---|
| Greatly improved | 3 |
| Improved | 2 |
| Somewhat improved | 1 |
| No effect | 0 |
| Deteriorated | -1 |

### (2) Ratings of effect for improving wrinkles and fine wrinkles

| | |
|---|---|
| Greatly improved | 3 |
| Improved | 2 |
| Somewhat improved | 1 |
| No effect | 0 |
| Deteriorated | -1 |

The test results are shown in Table 2B.

**Table 2B**

| | Tension/Sagging | Wrinkles/Fine wrinkles |
|---|---|---|
| Ex. 1B | 2.6 | 2.3 |
| Ex. 2B | 2.7 | 2.2 |
| Comp. Ex. 1B | 0.5 | 0.6 |

The results indicate that the groups using the agents of Examples 1B and 2B exhibit excellent improvement effect as compared to the group using the agent of Comparative Example 1B.

These four tests proves that the agent according to the present invention promotes the production of type-VII collagen, which is a component of extracellular matrix, to thereby activate extracellular matrix; normalize skin tissue; and reinforce the binding between the skin and the dermis. Thus, the agent according to the present invention effectively prevents skin aging.

A variety of formulation examples of the agent according to the present invention will next be disclosed. All of these production promoting agents exhibit effects for maintaining skin moisture; improving tension and sagging of the skin; and improving wrinkles and fine wrinkles at levels similar to those attained in the above actual application tests.

### [Formulation Example 1-1] Milky lotion (1)

| | amount (wt.%) |
|---|---|
| stearic acid | 6.0 |
| sorbitan ester of monostearic acid | 2.0 |
| polyoxyethylene (20 mol) sorbitan ester of monostearic acid | 1.5 |
| 2-(2'-hydroxy-5'-methylphenyl)benzotriazole | 8.0 |
| propylene glycol | 10.0 |
| Saiko extract | 10.0 |
| Preservative/antioxidant | suitable amount |
| perfume | suitable amount |
| purified water | balance |

### 〈Method of Preparation〉

Propyleneglycol was added to purified water and the resultant solution was heated and maintained at 70°C (aqueous phase). The remaining ingredients were mixed together, and the resultant mixture was heated so as to dissolve the ingredients. Thereafter, the temperature of the mixture was maintained at 70°C (oily phase). As a preliminary emulsification, the oily phase was added into the aqueous phase with stirring. The emulsion particles in the resultant mixture were finely divided and homogenized by use of a homomixer, followed by rapid cooling with vigorous stirring to thereby obtain a milky lotion.

### [Formulation Example 1-2] Milky lotion (2)

| | amount (wt.%) |
|---|---|
| stearic acid | 6.0 |
| sorbitan ester of monostearic acid | 2.0 |
| polyoxyethylene (20 mol) sorbitan ester of monostearic acid | 1.5 |
| 2-(2'-hydroxy-5'-methylphenyl)benzotriazole | 8.0 |
| propylene glycol | 10.0 |
| saikosaponin b2 | 1.0 |
| preservative/antioxidant | suitable amount |
| perfume | suitable amount |
| purified water | balance |

### 〈Method of Preparation〉

The procedure of Formulation Example 1-1 was repeated to thereby prepare milky lotion (2).

### [Prescription Example 2-1] Cream (1)

| | amount (wt.%) |
|---|---|
| polyoxyethylene (20 mol) cetyl ether | 1.0 |
| methylphenylpolysiloxane (20 cs) | 2.0 |
| liquid paraffin | 3.0 |
| 2-hydroxy-4-methoxybenzophenone | 5.0 |
| saikosaponin d | 0.3 |
| propylene glycol | 5.0 |
| glycerin | 2.0 |
| ethyl alcohol | 15.0 |
| carboxyvinylpolymer | 0.3 |
| hydroxypropylcellulose | 0.1 |
| 2-aminomethylpropanol | 0.1 |
| preservative | suitable amount |
| perfume | suitable amount |
| purified water | balance |

### 〈Method of Preparation〉

Propyleneglycol was added to purified water and the resultant solution was heated and maintained at 70°C (aqueous phase). The remaining ingredients were mixed together and the resultant mixture was heated so as to dissolve the ingredients. Thereafter, the mixture was maintained at 70°C (oily phase). As a preliminary emulsification, the oily phase was added into the aqueous phase with stirring. The emulsion particles in the resultant mixture were finely divided and homogenized by use of a homo-mixer, followed by rapid cooling with vigorous stirring to thereby obtain a cream.

### [Formulation Example 2-2] Cream (2)

| | amount (wt.%) |
|---|---|
| polyoxyethylene (20 mol) cetyl ether | 1.0 |
| methylphenylpolysiloxane (20 cs) | 2.0 |
| liquid paraffin | 3.0 |
| 2-hydroxy-4-methoxybenzophenone | 5.0 |
| saikosaponin d | 0.5 |
| propylene glycol | 5.0 |
| glycerin | 2.0 |
| ethyl alcohol | 15.0 |
| carboxyvinylpolymer | 0.3 |
| hydroxypropylcellulose | 0.1 |
| 2-aminomethylpropanol | 0.1 |
| preservative | suitable amount |
| perfume | suitable amount |
| purified water | balance |

### 〈Method of Preparation〉

The procedure of Formulation Example 2-1 was repeated to thereby prepare cream (2).

### [Formulation Example 3-1] Sunblock beauty lotion (1)

| | amount (wt.%) |
|---|---|
| stearic acid | 3.0 |
| cetyl alcohol | 1.0 |
| lanolin derivative | 2.0 |
| liquid paraffin | 2.0 |
| 2-ethylhexyl stearate | 2.0 |
| 1,3-butylene glycol | 6.0 |
| polyoxyethylene (10 mol) monooleate | 2.0 |
| glycerol monostearate | 2.0 |
| triethanolamine | 1.0 |
| 2-hydroxy-4-methoxybenzophenone | 3.0 |
| octyl p-methoxycinnamate | 2.0 |
| saikosaponin a | 0.05 |
| perfume | suitable amount |
| purified water | balance |

### 〈Method of Preparation〉

1,3-Butylene glycol and triethanolamine were dissolved in purified water, and the resultant solution was heated so as to maintain its temperature at 70°C (aqueous phase). The remaining ingredients were mixed together and the resultant mixture was heated so as to dissolve the ingredients. Thereafter, the mixture was maintained at 70°C (oily phase). As a preliminary emulsification, the oily phase was added into the aqueous phase with stirring. The emulsion particles in the resultant mixture were finely divided and homogenized by use of a homo-mixer, followed by rapid cooling with vigorous stirring to thereby obtain a sunblock beauty lotion.

### [Formulation Example 3-2] Sunblock beauty lotion (2)

| | amount (wt.%) |
|---|---|
| stearic acid | 3.0 |
| cetyl alcohol | 1.0 |
| lanolin derivative | 2.0 |
| liquid paraffin | 2.0 |
| 2-ethylhexyl stearate | 2.0 |
| 1,3-butylene glycol | 6.0 |
| polyoxyethylene (10 mol) monooleate | 2.0 |
| glycerol monostearate | 2.0 |
| triethanolamine | 1.0 |
| 2-hydroxy-4-methoxybenzophenone | 3.0 |
| octyl p-methoxycinnamate | 2.0 |
| saikosaponin a | 0.001 |
| perfume | suitable amount |
| purified water | balance |

### 〈Method of Preparation〉

The procedure of Formulation Example 3-1 was repeated to thereby prepare sunblock beauty lotion (2).

### [Formulation Example 4-1] Emulsion-type foundation (W/O emulsion-type) (1)

| | amount (wt.%) |
|---|---|
| organic compound-modified montmorillonite | 0.5 |
| cetyl isooctanate | 2.0 |
| octamethylcyclotetrasiloxane | 2.0 |
| decamethylcyclopentasiloxane | 5.0 |
| dimethylpolysiloxane (6 cs) | 5.0 |
| liquid paraffin | 3.0 |
| dioctadecyldimethylammonium chloride | 0.2 |
| polyoxyalkylene compound-modified organopolysiloxane | 5.0 |
| 4-tert-butyl-4'-methoxydibenzoylmethane | 0.3 |
| glyceryl mono-2-ethylhexanoyl di-p-methoxycinnamate | 1.0 |
| micro particle titanium oxide | 5.0 |
| oleyl alcohol | 0.5 |
| stearic acid | 0.5 |
| sorbitan diisostearate | 4.0 |
| antioxidant | suitable amount |
| perfume | suitable amount |
| talc | 1.5 |
| nylon powder | 1.0 |
| purified water | balance |
| sodium citrate | 0.5 |
| 1,3-butylene glycol | 5.0 |
| saikosaponin b2 | 0.5 |

### 〈Method of Preparation〉

Sodium citrate, 1,3-butylene glycol, saikosaponin b2, and an antioxidant were added to purified water, and the resultant solution was stirred with heat at 70°C (aqueous phase). Micro particle titanium oxide, talc, and nylon powder were sufficiently mixed and crushed (powder portion). The remaining ingredients were mixed together and the resultant mixture was heated and melted. Thereafter, the mixture was maintained at 70°C (oily phase). The powder portion was added into the aqueous phase, and resultant mixture was processed by use of a homo-mixer at 70°C. The oily phase was added thereto, and the emulsion particles in the resultant mixture were finely divided and homogenized by use of the homo-mixer, followed by rapid cooling with vigorous stirring to thereby obtain an emulsion-type foundation.

### [Formulation Example 4-2] Emulsion-type foundation (W/O emulsion-type) (2)

| | amount (wt.%) |
|---|---|
| organic compound-modified montmorillonite | 0.5 |
| cetyl isooctanate | 2.0 |
| octamethylcyclotetrasiloxane | 2.0 |
| decamethylcyclopentasiloxane | 5.0 |
| dimethylpolysiloxane (6 cs) | 5.0 |
| liquid paraffin | 3.0 |
| dioctadecyldimethylammonium chloride | 0.2 |
| polyoxyalkylene compound-modified organopolysiloxane | 5.0 |
| 4-tert-butyl-4'-methoxydibenzoylmethane | 0.3 |
| glyceryl mono-2-ethylhexanoyl di-p-methoxycinnamate | 1.0 |
| micro particle titanium oxide | 5.0 |
| oleyl alcohol | 0.5 |
| stearic acid | 0.5 |
| sorbitan diisostearate | 4.0 |
| antioxidant | suitable amount |
| perfume | suitable amount |
| talc | 1.5 |
| nylon powder | 1.0 |
| purified water | balance |
| sodium citrate | 0.5 |
| 1,3-butylene glycol | 5.0 |
| Saiko extract | 5.0 |

### 〈Method of Preparation〉

The procedure of Formulation Example 4-1 was repeated to thereby prepare emulsion-type foundation (W/O Emulsion-type) (2).

### [Formulation Example 5-1] Powdery foundation (1)

| | amount (wt.%) |
|---|---|
| micro particle titanium oxide | 7.0 |
| talc | 40.0 |
| mica | balance |
| nylon powder | 10.0 |
| red iron oxide | 1.0 |
| yellow iron oxide | 2.0 |
| black iron oxide | 0.2 |
| dimethylpolysiloxane | 1.0 |
| 2-ethylhexyl palmitate | 9.0 |
| sorbitan sesquioleate | 1.0 |
| octyl N,N-dimethyl PABA | 0.3 |
| saikogenin A | 0.2 |
| preservative | suitable amount |
| antioxidant | suitable amount |
| perfume | suitable amount |

### 〈Method of preparation〉

Micro particle titanium oxide, talc, mica, nylon powder, red iron oxide, yellow iron oxide, black iron oxide, and saikogenin A were sufficiently crushed and mixed by use of a blender (powder portion). To the powder potion, dimethylpolysiloxane, 2-ethylhexyl palmitate, sorbitan sesquioleate, octyl N,N-dimethyl PABA, a preservative, and an antioxidant were added and thereafter a perfume was sprayed onto the resultant mixture. The mixture was homogeneously mixed, crushed by use of a crusher, introduced into a mold, and molded to thereby obtain a powdery foundation.

### [Formulation Example 5-2] Powdery foundation (2)

| | amount (wt.%) |
|---|---|
| micro particulate titanium oxide | 7.0 |
| talc | 40.0 |
| mica | balance |
| nylon powder | 10.0 |
| red iron oxide | 1.0 |
| yellow iron oxide | 2.0 |
| black iron oxide | 0.2 |
| dimethylpolysiloxane | 1.0 |
| 2-ethylhexyl palmitate | 9.0 |
| sorbitan sesquioleate | 1.0 |
| octyl N,N-dimethyl PABA | 0.3 |
| saikogenin A | 5.0 |
| preservative | suitable amount |
| antioxidant | suitable amount |
| perfume | suitable amount |

### 〈Method of preparation〉

The procedure of Formulation Example 5-1 was repeated to thereby prepare powdery foundation (2).

### [Formulation Example 6-1] Oily foundation (1)

| | amount (wt.%) |
|---|---|
| micro particle titanium oxide | 10.0 |
| mica | 22.4 |
| kaolin | 10.0 |
| nylon powder | 5.0 |
| red iron oxide | 0.5 |
| yellow iron oxide | 0.5 |
| black iron oxide | 0.1 |
| liquid paraffin | balance |
| dimethylpolysiloxane | 10.0 |
| sorbitan sesquioleate | 2.0 |
| octyl methoxycinnamate | 5.0 |
| saikogenin D | 0.01 |
| perfume | suitable amount |
| microcrystalline wax | 6.0 |
| carnauba wax | 3.0 |

### 〈Method of preparation〉

Micro particle titanium oxide, mica, kaolin, nylon powder, red iron oxide, yellow iron oxide, and black iron oxide were sufficiently mixed and crushed by use of a blender (powder portion). The remaining ingredients other than a perfume were mixed together, and the resultant mixture was melted by heating at 85°C (oily phase). The powder potion was added into the oily phase with stirring. A perfume was added to the resultant mixture. The mixture was sufficiently ground and dispersed, introduced into a mold, and molded to thereby obtain an oily foundation.

### [Formulation Example 6-2] Oily foundation (2)

| | amount (wt.%) |
|---|---|
| micro particle titanium oxide | 10.0 |
| mica | 22.4 |
| kaolin | 10.0 |
| nylon powder | 5.0 |
| red iron oxide | 0.5 |
| yellow iron oxide | 2.0 |
| black iron oxide | 0.1 |
| liquid paraffin | balance |
| dimethylpolysiloxane | 10.0 |
| sorbitan sesquioleate | 2.0 |
| octyl methoxycinnamate | 5.0 |
| saikogenin D 0 | 0.005 |
| perfume | suitable amount |
| microcrystalline wax | 6.0 |
| carnauba wax | 3.0 |

### 〈Method of preparation〉

The procedure of Formulation Example 6-1 was repeated to thereby prepare oily foundation (2).

### Industrial Applicability

The agent according to the present invention promotes the production of extracellular matrix; specifically, acidic mucopolysaccharides such as hyaluronic acid and fibrous proteins such as collagen, to thereby activate extracellular matrix; normalize skin tissue; and reinforce the binding between the epidermis and the dermis. Thus, the agent according to the present invention effectively prevents skin aging.

## Claims

1. An extracellular matrix production promoting agent comprising an extract of a plant belonging to the genus *Bupleurum* as an active ingredient.

2. An extracellular matrix production promoting agent according to claim 1, wherein the source of the extract of a plant belonging to the genus *Bupleurum* is root portion of the plant.

3. An extracellular matrix production promoting agent according to claim 1 or 2, wherein the plant belonging to the genus *Bupleurum* is *B. falcatum Linne.*

4. An extracellular matrix production promoting agent comprising saikosaponin and/or saikogenin as an active ingredient.

5. An extracellular matrix production promoting agent according to any one of claims 1 through 4, which is a skin treatment preparation for external use.

6. An extracellular matrix production promoting agent according to any one of claims 1 through 5, which promotes production of an acidic mucopolysaccharide and a fibrous protein.

7. An extracellular matrix production promoting agent according to any one of claims 1 through 5, which promotes production of an acidic mucopolysaccharide.

8. An extracellular matrix production promoting agent according to any one of claims 1 through 5, which promotes production of a fibrous protein.

9. An extracellular matrix production promoting agent according to claim 6 or 7, wherein the acidic mucopolysaccharide is hyaluronic acid.

10. An extracellular matrix production promoting agent according to claim 6 or 8, wherein the acidic mucopolysaccharide is collagen.
